Europäisches Patentamt

European Patent Office

Office européen des brevets

(19)

(11) Veröffentlichungsnummer: **0 162 129**
**A1**

(12)

# EUROPÄISCHE PATENTANMELDUNG

(21) Anmeldenummer: 84111391.3

(22) Anmeldetag: 25.09.84

(51) Int. Cl.⁴: **A 61 K 9/50**
**A 61 K 47/00**

(30) Priorität: 11.04.84 DE 3413541
13.09.84 DE 3433609

(43) Veröffentlichungstag der Anmeldung:
27.11.85 Patentblatt 85/48

(84) Benannte Vertragsstaaten:
AT BE CH FR GB IT LI LU NL SE

(71) Anmelder: Theurer, Karl Eugen, Prof.Dr.med.
Brunnwiesenstrasse 23
D-7302 Ostfildern 1(DE)

(72) Erfinder: Theurer, Karl Eugen, Prof.Dr.med.
Brunnwiesenstrasse 23
D-7302 Ostfildern 1(DE)

(54) Herstellung von Arznei- und Diätmittel aus Milch, Sahne oder Butterfett und deren Verwendung.

(57) Fettmikronen aus Milch oder Sahne, wie auch Butter unter Einbeziehung von behannten Faktoren von künstlichen und bzw. oder Zellmembranen und bzw. oder Tracer-Molekülen zu Target-Zellen und bzw. oder lipidlösliche Pharmaka werden zur Herstellung von Liposomen oder Membranfragmenten mit inkorporierten Pharmaka als Diät- oder Arzneimittel verwendet, wobei in der äußeren, wässrigen Phase der Doppelemulsion andersartiger Arzneimittel als in den Liposomen bzw. Liposomenmembranen enthalten sein können und die Milchproteine bzw. -saccharide durch Arzneimittellösungen ergänzt oder ersetzt werden.

EP 0 162 129 A1

Croydon Printing Company Ltd.

Herstellung von Arznei- und Diätmittel aus Milch,
Sahne oder Butterfett und deren Verwendung.


Beschreibung:

Das Verfahren betrifft die Herstellung von Arznei-
und Diätmittel aus Milch, Sahne oder Butterfett mit
biologischen Wirkfaktoren aus Mikroorganismen,
Pflanzen oder xenogenen Organgeweben in Form von Proteinen und bzw. oder deren Untereinheiten und Bestandteilen wie Peptide, Nukleotide, Nukleoside, wie auch
andersartigen, wasserlöslichen Pharmaka, einzeln oder
in Mischung zur Ergänzung oder zum Ersatz der Milcheiweiße bzw. -saccharide. Die biologischen Wirkfaktoren
und bzw. oder Arzneimittel werden durch Homogenisieren
oder Einwirkung von Ultraschall in Liposomen aus Milch-
oder Butterfett inkorporiert.

Speziell für die Gewinnung von Arzneimitteln wird das
Verfahren weiter ausgestaltet durch die Verwendung von
Lipiden aus Milch, Sahne oder Butter, zusammen mit
Cholesterin und Phospholipiden bzw. Faktoren von Zellmembranen wie sie bisher zur Gewinnung von Liposomen
und künstlichen Membranen verwendet werden. Dabei können zusätzliche Faktoren mit einem Tropismus zu Target-
Zellen als Tracer-Moleküle (vergleiche Theurer:
DP 2650502 und DP 3339907.7) sowie lipidlösliche Arzneimittel in die Liposomenmembran eingebaut oder an
diese kovalent oder adsorptiv gebunden werden. Kohlehydrate und Proteine der Milch oder Sahne können mit
bekannten Methoden beseitigt und einer andersartigen
Verwendung zugeführt werden.

Liposomen sind kleinste Fettbläschen mit wässrigem Inhalt im Zentrum. Diese entstehen durch Homogenisieren oder Ultraschalleinwirkung aus Fettmikronen. Die Herstellungsverfahren sind bekannt, z.B. HEYDON und TAYLOR: J. Theoret. Biol. 4, 281 (1963); MUELLER, FUDIN, TITIEN, WESTCOTT: Nature 194, 979 (1962); GREGORIADES: FEDS Letters 36, 3 (1973); BANGHAM: Progr. Biophys. Mol. Biol. 18, 19 (1968) und RAHMANN, ROSENTHAL und CERNY: Science 180, 300 (1973): J. Lab. Klin. Med. 83, 640 (1974); Procedings of Society for expermental Biology and Medicine 146, 1173-1176 (1974). Bei THEURER K.E.: DP 2650502 vom 4.11.1976 und Zusatzpatent DP 3339907.7 werden Enzyme, zellspezifische Immunglobuline oder spezifische Faktoren, die den Target-Zellen entsprechen, aus Zell- und Gewebeextrakten bzw. daraus isolierten Membranen an die Liposomenmembran bzw. an Membranbestandteile chemisch kovalent oder adsorptiv gebunden, um einen Zelltropismus mit verbesserter Adsorption zu erreichen.

Liposome wurden als Träger für die verschiedensten Arzneimittel, Enzyme, Eiweißlösungen, wie auch zum Transport von Nukleinsäuren und deren Untereinheiten gebraucht (vergl. TIBS, Sept. 1976, S. 203 ff und FEBS Letters, Febr. 1976.

Die Verwendung von Milch und Sahne mit z.B. 30-50 % Fettanteil, in Verbindung mit zugesetztem löslichen Eiweiß, und bzw. oder Nukleinsäuren und bzw. oder deren Untereinheiten und wässrige Bestandteile in wässrigen Lösungen ist bisher nicht erfolgt, obwohl Milch zum Zwecke der Haltbarmachung und Konservierung ohne Zusätze seit langem homogenisiert wird
(H-Milch als Vollmilch oder Magermilch). Auch die
erfindungsgemäßen Produkte sind bei Verwendung
steriler Ausgangsmaterialien gut haltbar.

Es werden frische oder konservierte biologische
Wirkfaktoren inkorporiert. Aus Mikroorganismen können Vaccine oder sterile Autolysate aus Kulturen aus
physiologischen Bakterienarten, wie z.B. Escherichia,
Coli, Streptococcos faecalis, Lactobacterium bifidum
u.a. verwendet werden, ebenso Stoffwechselprodukte
von Milchsäurebildnern. Auch aus Viren können nicht
infektiöse Bestandteile, wie z.B. isolierte Eiweißanteile oder Nukleotide verwendet werden. Als pflanzliche Wirkfaktoren können solche aus Pflanzenpreßsäften und allgemein lösliche und haltbare Phytotherapeutika verwendet werden.

Faktoren aus Organgeweben mit Zelltropismus ermöglichen
eine Vehikelfunktion zu korrespondierenden Geweben,
wenn sie in die Lipidmembran eingebaut werden. Sonst
können aber die beanspruchten Wirkfaktoren aus unterschiedlichen Organarten einzeln oder als Mischungen
über die Liposome zur Wirkung gebracht werden. Es handelt sich bei den Wirkfaktoren um Biological Response
Modifiers (vergl. PORCHER, H.: Med. Klin. 77, Nr. 6,
10-12 (1982) sowie THEURER, K.E.: Pharmakologie: Eingliederung der Therapie mit makromolekularen Organextrakten in die moderne Pharmakologie: DER KASSENARZT
21, 12 (1981), sowie Aktivierung von Selbstheilungsvorgängen - Hygiogenese durch Organo- und Immunotherapie: Der prakt. Tierarzt H. 6 und 7 (1982). Es besteht
die Möglichkeit der verschiedenartigsten Kombination von Faktoren, auch als Mischung von Mikroorganismen und pflanzlichen Bestandteilen und Organfaktoren.

Die Zusatzstoffe können als wässrige Lösungen zur Milch oder Sahne zugesetzt werden. Auch ist es möglich, Trockenprodukte zu verwenden. Die Lösungen derselben erfolgt zweckmäßig mit Milch, wie z.B. die Sol-Präparate von Revitorgan[R]. Die Konzentrationen der Zusätze liegt im mg- bis pg-Bereich. Normalerweise werden Mengen zugesetzt, die eine Konzentration im Fertigprodukt von µg bis ng ermöglichen.

Es können alle Arten von wasserlöslichen und nicht lipidlöslichen Pharmaka in die Liposome inkorporiert werden, insbesondere Hormone, Vitamine, Spurenelemente wie auch chemische Pharmaka der verschiedensten Art. Lipidlösliche Pharmaka lassen sich zusätzlich zu Tracer-Substanzen der Lipid-Kombination für die Liposomenmembran verwenden. Der Anteil von Milch- oder Butterfett beträgt bis zu 40%: die Menge der Arzneimittel bis zu 5% der Lipidmischung. Die jeweils optimale Methode ist sowohl von der Art der Liposomenmembran, wie auch von der jeweils in die Membran zu inkorporierenden oder anzukoppelnden Substanz abhängig. Fragmente von Liposomenmembranen werden in wässrigen Arzneimittellösungen emulgiert. Die Applikation kann sowohl lokal als auch systemisch erfolgen.

Die wässrige Phase der Milch, Sahne oder Butter kann durch wässrige Arzneimittellösungen ergänzt oder ausgetauscht werden. Auch lassen sich in der äußeren, wässrigen Phase andersartige Arzneimittel verwenden, als diejenigen, die in den Liposomen inkorporiert sind. Es muß dann nach Herstellung der Liposomen die Arzneimittellösung, die bei der Herstellung der Liposomen verwendet wurde, gegen eine andere ausgetauscht werden. In den Liposomen und Membranfragmenten selbst können dann zusätzlich noch andere Pharmaka neben den Tracerstoffen Verwendung finden. Es gibt deshalb verschiedenartige Kombinationsmöglichkeiten. Bei chronischen Erkrankungen des Darmkanals (Colitis ulcerosa, Morbus Crohn) sowie dem intestinalen Strahlensyndrom werden bei Präparaten für die orale Applikation in der äußeren wässrigen Phase nicht oder schlecht resorbierbare, lösliche antiinfektiöse Stoffe wie z.B. Salazopyrin und in der inneren Phase der Liposomen Peptide aus Darmschleimhaut, Leber und Thymus gegebenenfalls zusammen mit Glukokortikoiden und in den Liposomenmembranen zytotrope Faktoren aus Darmschleimhaut oder Antikörper bzw. Antikörperfragmente auch aus geklonten Antikörpern, die organspezifisch gegen Darmschleimhaut gerichtet sind, verwendet. Als therapeutischer Faktor lassen sich in der Liposomenmembran zusätzlich Lipide aus Zellmembranen analog der geschädigten Gewebeart wie auch lipidlösliche Pharmaka einsetzen. Zur Stabilisierung der Emulsion besonders aus Lipidmembranen, sind, wie auch von Liposomen- Suspensionen allgemein bekannte Emulgatoren geeignet.

Beispiel 1:
Es soll ein Diät-Präparat für Magen-Darm-Störungen aus Vollmilch hergestellt werden. Dazu wird eine Organkombination, entsprechend den zytoplasmatischen Organ- Präparaten der Firma vitOrgan Arzneimittel GmbH, D-7302 Ostfildern-Ruit, verwendet. Dieses Präparat ist auf 15 mg voll-löslicher Trockensubstanz eingestellt und enthält Faktoren mit Molekulargewicht zwischen 1.000 und 1 Million einer Mischung von Proteinen und Nukleinsäuren sowie deren Untereinheiten. Es werden davon 10 mg in 10 1 Milch gelöst, sodaß die Endkonzentration 1 µg pro ml ($10^{-6}$) beträgt. Anschließend wird die Milch mit einer Schall-Leistung von 120-240 W bei einer Betriebsfrequenz von 35 KHz während einer halben Stunde beschallt und danach in üblicher Weise für den Verbrauch in geeigneten Mengen abgefüllt.

Beispiel 2:
Herstellung von Präparaten aus Mikroorganismen. Hier werden nicht infektiöse Extrakte aus Bakterien und bzw. oder Viren , in gleicher Weise wie in Beispiel 1, verarbeitet. Auch ist eine Kombination mit Organextrakten möglich. Die Endkonzentration der Extrakte aus Mikroorganismen liegt im pg-Bereich pro ml. Es müssen deshalb zu 10 1 Milch 10 ng Wirkstoff zugesetzt werden.

Beispiel 3:
Phytotherapeutische Faktoren werden in gleicher Art wie in Beispiel 1 und 2 - aufgrund pharmakologischer Erkenntnisse - einzeln oder in Kombination mit Mikroorganismen oder Organfaktoren verwendet.

Beispiel 4:
Es soll ein roborierendes Diät-Präparat aus Sahne hergestellt werden. Dazu wird eine Organmischung wie in REVITORGAN[R] NeyGeront[R] , in gleicher Weise wie in Beispiel 1, verwendet.

Beispiel 5:
Es soll ein Präparat zur Stimulierung des Sympathikotonus mit katabolisierender Wirkung aus Vollmilch hergestellt werden. Hierzu wird eine Organkombination, wie in NeyDop[R] (REVITORGAN[R]Nr. 97), in einer Endkonzentration von $10^{-9}$ verwendet. Zusätzlich können Einzelorganextrakte, wie z.B. Thyreoglobulin aus Schilddrüse, mitverwendet werden.

Beispiel 6:
Es soll ein Arzneimittel zur Prophylaxe der Pollenallergie unter Verwendung von Butter hergestellt werden. Dazu wird frische oder gefrorene Butter zusammen
mit Ei- oder Sojalecithin und Cholesterin im Verhältnis 3:5:2 Teile als Mischung durch Erwärmen beim
Schmelzpunkt, möglichst unter Ausschluß von Sauerstoff,
gegebenenfalls durch Abdecken der Schmelze mit chemisch
inerten Gasen und unter Druck von über 1 Atmosphäre
durch eine Düse in eine wässrige Arzneimittellösung
versprüht, sodaß sich eine Emulsion aus Fettröpfchen
und Liposomen bildet.

Dem Lipidgemisch kann ein Extrakt aus Proteinen, Peptiden, Nukleotiden oder Lipiden aus Schleimhautzellen
und bzw. oder aus lymphatischen Zellen und Makrophagen in der Endkonzentration bis zu 1% der Lipidbestandteile zugesetzt werden.

Als Arzneilösung wird die wässrige Lösung eines Gemisches verschiedener Pollenantigene die zur Allergie
führen können, gegebenenfalls mit Zusatz von Theophyllin (1,3-Dimethylxanthin) in einer Konzentration
bis zu 0,2 g/ml Lösungsmittel und bzw. oder Diphenylmethylpiperazin, (1-(3-(4-Diphenylmethyl)-1-Peperazinyl)-
prophyl)-1,3-dihydro-2H-benzimidazol-2-on) in einer
Konzentraion bis zu 30 mg/ml Lösungsmittel, benutzt.
Zur weiteren Ausbildung von Liposomen wird die Emulsion bzw. Suspension der Einwirkung von Ultraschall
bei einer Leistung von 100-200 W und einer Betriebsfrequenz von 35 KHz während 15 Minuten ausgesetzt.

Die äußere wässrige Phase der Allergen- bzw. Arzneimittellösung wird durch bekannte Methoden der Molekularfiltration bzw. -zentrifugation bei Spülen der Liposomenfraktion mit isotoner physiologischer Lösung entfernt und gegebenenfalls erneut verwendet. Dem Suspensionsmittel der Liposome wird eine Mischung aus wasserlöslichen Organextrakten aus Nebenniere, foetalem Thymus und foetaler Plazenta vom Rind in einer Endkonzentration von ng/ml Lösungsmittel zugesetzt. Die Applikation des Präparates kann in Form eines Aerosols durch Inhalation oder oral erfolgen.

## Beispiel 7:

Frischmilch wird von Eiweiß und Sacchariden durch bekannte Methoden (Elektrophorese, Dialyse, Flüssigkeitschromatographische Verfahren, Aussalzen mit Ammoniumsulfat, Ausfällen mit Trichloressigsäure oder Abrahmen) befreit und die wässrige Lösung durch eine Arzneilösung, in welcher die Fettbestandteile resuspendiert oder -emulgiert werden, ersetzt. Durch Homogenisieren oder Ultraschalleinwirkung werden aus den Fettmikronen Liposome hergestellt, in denen die Arzneilösung inkorporiert ist. Durch Filtration bei Überströmtechnik wird das Suspensionsmittel gegen eine andere Art von wässriger Arzneimittellösung ausgetauscht, zum Beispiel durch die Lösung einer nicht resorbierbaren bakteriostatisch oder bakteriozid wirkenden Substanz.

0162129

Beispiel 8:

Es werden Frischmilch oder -sahne eine Arzneimittellösung, z.B. von Insulin, von Wachstumshormonen, von
Somatotropin, von Cortison, von einem Herzglykosid
oder von irgendeinem anderen Arzneimittel in geeigneter Menge und Konzentration zugesetzt, bevor Liposome
durch Homogenisieren hergestellt werden. Die wässrige
Lösung der äußeren Phase kann durch physiologische
Lösungen verdünnt oder aber zu erneuter Verwendung
wie in Beispiel 2 gegen eine andersartige Arzneimittellösung bzw. physiologische Lösung ausgetauscht
werden. Nach Inkorporierung in Liposome können die
Arzneimittelzusätze auch durch Gegenstoffe inaktiviert sowie ausgefällt oder beseitigt werden.

Beispiel 9:

Die in P 3413541.3 angeführten Beispiele 1-5 dienen
bei Anwendung von mehr als 10-fach höheren Konzentrationen und Dosierungen der zugesetzten Wirkstoffe zur
Herstellung von Arzneimitteln.

Herstellung von Arznei- und Diätmittel aus Milch,
Sahne oder Butterfett und deren Verwendung.


Patentansprüche:

1.  Herstellung von Arznei- und Diätmittel aus Milch,
    Sahne oder Butterfett durch Homogenisieren bzw.
    Behandlung mit Ultraschall,
    dadurch gekennzeichnet, daß vor dem Homogenisieren biologisch wirksame Faktoren aus Mikroorganismen, Pflanzen oder xenogenen Organgeweben in Form
    von Proteinen und bzw. oder Nukleinsäuren und bzw.
    oder deren Untereinheiten und Bestandteile (Peptide,
    Nukleotide, Nukleoside) wie auch andersartige, wasserlösliche Pharmaka, einzeln oder in Mischung als
    Ergänzung oder Ersatz der Milcheiweiße und bzw.
    oder -saccharide der wässrigen Phase zugesetzt und
    in Liposome aus Fettmikronen inkorporiert werden,
    wobei die äußere wässrige Phase durch andersartige
    Arzneimittellösungen als im Inneren der Liposomen
    ersetzt und der Liposomenmembran Tracer-Substanzen
    mit Zelltropismus zu Target-Zellen oder lipidlösliche
    Pharmaka kovalent oder adsorptiv angekoppelt oder
    eingelagert sein können und solche Liposomenmembranen auch zu einer Lipid-in-Wasser-Emulsion mit
    zusätzlichen Arzneimitteln in der wässrigen Phase
    verwendet werden.


2.  Verfahren nach Anspruch 1,
    dadurch gekennzeichnet, daß Milch- oder Butterfett
    zusammen mit bekannten Membranfaktoren, insbesondere
    Phospholipiden und Cholesterin zu Liposomen oder
    Membranfragmenten verarbeitet werden.

3.  Verfahren nach Anspruch 1 und 2,
    dadurch gekennzeichnet, daß in der äußeren wässrigen Phase Antibiotika oder antimikrobielle bzw.
    -virale Chemotherapeutika und in den Liposomen
    zellreparative und -membranstabilisierende Pharmaka
    verwendet werden.

4.  Verfahren nach Anspruch 1-3,
    dadurch gekennzeichnet, daß lösliche Allergene
    einzeln oder als Mischung in die Liposome inkor-
    poriert werden.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-B-1 767 447   (PREPHAR) <br> * Insgesamt * | 1 | A 61 K   9/50 <br> A 61 K  47/00 |
| Y | . | 2,4 | |
| | --- | | |
| Y | DE-A-2 650 502   (THEURER) <br> * Insgesamt * | 2 | |
| | --- | | |
| A | FR-M-   3 789  (SOCIETE INDUSTRIELLE POUR LA FABRICATION DES ANTIBIOTIQUES) <br> * Seite 3, Beispiel * | | |
| | --- | | |
| Y | EP-A-0 017 557   (MERCK & CO.) <br> * Ansprüche * | 4 | |
| | ----- | | |

| RECHERCHIERTE SACHGEBIETE (Int. Cl.4) |
|---|
| A 61 K <br> A 23 C |

Der vorliegende Recherchenbericht wurde für alle Patentansprüche erstellt.

| Recherchenort <br> DEN HAAG | Abschlußdatum der Recherche <br> 15-07-1985 | Prüfer <br> BENZ K.F. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82